# EUROPÄISCHE PATENTSCHRIFT

(11) **EP 2 391 963 B1**
(45) Veröffentlichungstag und Bekanntmachung des Hinweises auf die Patenterteilung: **21.06.2017**
(21) Anmeldenummer: 11721988.1
(22) Anmeldetag: 02.03.2011
(51) Int. Cl.: G06F 19/00, G09B 23/28

(54) **VERFAHREN ZUR DURCHFÜHRUNG EINER VIRTUELLEN OPERATION ZU TRAININGSZWECKEN**
METHOD FOR CARRYING OUT A VIRTUAL OPERATION FOR TRAINING PURPOSES
PROCÉDÉ POUR RÉALISER UNE OPÉRATION VIRTUELLE AUX FINS D'ENTRAÎNEMENT

(30) Priorität: 02.03.2010 DE 102010010002
(43) Veröffentlichungstag der Anmeldung: 07.12.2011
(73) Patentinhaber: Geuder AG, 69126 Heidelberg (DE); VRmagic GmbH, 68165 Mannheim (DE)
(72) Erfinder: GEUDER, Volker, 69126 Heidelberg (DE); FRAUENFELD, Dieter, 69126 Heidelberg (DE); WAGNER, Clemens, 69115 Heidelberg (DE); SCHILL, Markus, 69117 Heidelberg (DE)
(74) Vertreter: Patent- und Rechtsanwälte Ullrich & Naumann
(86) Internationale Anmeldenummer: PCT/DE2011/000236
(87) Internationale Veröffentlichungsnummer: WO 2011/107088

(56) Entgegenhaltungen:
- WO-A2-2009/144745
- US-A1- 2007 207 448
- KHALIFA Y.M. ET AL: "Virtual Reality in Ophthalmology Training", SURVEY OF OPHTHALMOLOGY, Bd. 51, Nr. 3, 1. Mai 2006 (2006-05-01), Seiten 259-273, XP002647063, ISSN: 0039-6257, DOI: DOI:10.1016/J.SURVOPHTHAL.2006.02.005 [gefunden am 2006-05-01]
- MARKUS A SCHILL ET AL: "EyeSi - A Simulator for Intra-ocular Surgery", 1. Januar 2006 (2006-01-01), MEDICAL IMAGE COMPUTING AND COMPUTER-ASSISTED INTERVENTION - MIC CAI'99 LECTURE NOTES IN COMPUTER SCIENCE;;LNCS, SPRINGER, BERLIN, DE, PAGE(S) 1166 - 1174, XP019036146, ISBN: 978-3-540-66503-8 das ganze Dokument

## Beschreibung

Die Erfindung betrifft ein Verfahren zur Durchführung einer virtuellen Operation zu Trainingszwecken, insbesondere eines virtuellen ophthalmologischen Eingriffs, wobei das Verfahren über ein Operationsprogramm definiert ist, welches über einen Computer, vorzugsweise über einen PC, von einer Trainingsperson genutzt wird.

Bislang ist es üblich, dass Medizinstudenten bzw. angehende Ärzte, Assistenzärzte oder aber auch erfahrene Ärzte, die sich neue Operationsmethoden aneignen wollen, an Leichen, an Tieren oder an körperlichen Dummys spezifische Eingriffe üben. Dies ist in Bezug auf den Trainingseffekt unzureichend. Außerdem lassen sich von ein und derselben Trainingsperson wohl kaum beliebig viele Trainingseinheiten absolvieren, nämlich aufgrund der mit einer solchen Trainingseinheit verbundenen Kosten. Schließlich können die gewünschten Trainingseinheiten nur zu bestimmten Zeiten genommen werden, nämlich entsprechend dem jeweiligen Angebot.

Des Weiteren gibt es auch bereits EDV-Trainingsprogramme, wonach verschiedenartige Eingriffe am menschlichen Körper nachgeahmt oder anhand von Originalaufnahmen dargestellt sind. Praxisnahe Erfahrungen können mit Hilfe solcher Programme kaum vermittelt werden, da die Trainingsperson nur passiv beteiligt ist, allenfalls einzelne Operationsphasen wiederholen und dabei genauer betrachten kann. Ein Trainingseffekt kann dadurch nicht erreicht werden.

Aus dem Dokument US 2007/0207448 A1 ist ein Verfahren zur Durchführung eines virtuellen ophthalmologisches Eingriffs zu Trainingszwecken vorbekannt. Der Artikel Khalifa Y. M. et al.: "Virtual Reality in Ophthalmology Training", Survey of Ophthalmology, Bd 51, Nr.3, 1 Mai 2006, Seiten 259-273 gibt eine Übersicht über bekannte Verfahren und Vorrichtungen zur Durchführung von virtuellen Operationen zu Trainingszwecken in der Ophthalmologie. Die Veröffentlichung Markus A. Schill et al.: "EyeSi - A Simulator for Intra-ocular Surgery", Medical Image Computing and Computer Assisted Interventions - MIC CAI 99 Lecture Notes in Computer Science beschreibt einen Simulator zur Durchführung virtueller ophthalmologischer Eingriffe.

Des Weiteren ist aus der WO 2009/144745 A2 ein Verfahren zum Unterrichten von Schülern außerhalb einer Schule bekannt.

Der vorliegenden Erfindung liegt daher die Aufgabe zugrunde, ein Verfahren zur Durchführung einer virtuellen Operation anzugeben, welches sich zu Trainingszwecken eignet, insbesondere zur Durchführung eines virtuellen ophthalmologischen Eingriffs.

Erfindungsgemäß handelt es sich hier um ein Verfahren zur Durchführung einer virtuellen Operation zu Trainingszwecken, insbesondere zur Durchführung eines virtuellen ophthalmologischen Eingriffs, wobei das Verfahren über ein Operationsprogramm definiert ist und diverse Verfahrensschritte umfasst, die über einen Computer, vorzugsweise über einen PC, von einer Trainingsperson dezentral, d.h. individuell am PC, abarbeitbar sind. Unterbrechungen bzw. Pausen sind bei der Abarbeitung des Programms möglich. Das Verfahren umfasst folgende Verfahrensschritte:
- Darstellung eines Operationsfeldes am menschlichen oder tierischen Körper, insbesondere eines Auges, auf einem Display,
- virtuelle Vornahme eines spezifischen Eingriffs mit mindestens einem einem konkreten medizinischen Operationsgerät, ggf. mit apparativer Peripherie, nachgeahmten Dummy, wobei der Dummy bzw. die Bewegung und die Betätigung des Dummy in das Operationsfeld hinein projiziert werden,
- interaktive Darstellung der Operationsschritte bzw. des Operationsergebnisses auf dem Display,
wobei das Operationsprogramm über einen Datenträger oder über das Internet bereitgestellt wird und anhand einer Zugangsberechtigung von dem Datenträger ladbar bzw. über das Internet herunterladbar und aktivierbar ist.

Der vorliegenden Erfindung liegt die grundsätzliche Idee zugrunde, dass beliebige Operationen aus unterschiedlichsten medizinischen Disziplinen von der Trainingsperson virtuell durchführbar sind, wobei dazu ein konkretes Operationsfeld am menschlichen oder tierischen Körper, beispielsweise das Operationsfeld eines Auges, auf einem Display dargestellt wird. Im Konkreten kann es sich dabei um das Display eines PC oder eines Labtops/Notebooks handeln. So lässt sich beispielsweise eine Catarakt-Operation am menschlichen Auge simulieren, wobei ein solcher virtueller spezifischer Eingriff mit mindestens einem einem konkreten medizinischen Operationsgerät nachgeahmten Dummy erfolgt, der sich im Besitz der Trainingsperson befindet. Der Dummy bzw. die Bewegung und die Betätigung des Dummys werden rechnerisch in das Operationsfeld hinein projiziert, wobei dabei Methoden Anwendung finden, wie sie beispielsweise aus den Druckschriften DE 102 57 624 A1, DE 102 57 625 A1, DE 10 2007 023 506 A1, DE 10 2007 057 208 A1 und EP 1 025 520 B1, für sich gesehen bekannt sind. Die dort gezeigten Verfahren sind hier in vorteilhafter Weise anwendbar, wobei die EP 1 025 520 B1 u.a. auch eine Vorrichtung offenbart, die im Sinne des hier als Dummy bezeichneten Handgeräts mit den erforderlichen Grundfunktionen verwendbar ist.

Ein entsprechendes Handgerät zur Durchführung einer virtuellen Operation ist für sich gesehen aus der DE 10 2007 045 875 A1 bekannt.

Es erfolgt eine interaktive Darstellung der Operationsschritte bzw. des Operationsergebnisses auf dem Display, so beispielsweise bis hin zum Einsatz einer künstlichen Vorderkammerlinse oder einer entsprechenden Hinterkammerlinse, die in den vom natürlichen Linsenkörper befreiten, dort verbliebenen Kapselsack eingesetzt worden ist.

Das Operationsprogramm wird über einen der Trainingsperson zur Verfügung gestellten Datenträger oder über das Internet bereitgestellt und ist anhand einer Zugangsberechtigung von dem Datenträger ladbar bzw. über das Internet herunterladbar und entsprechend aktivierbar.

In vorteilhafter Weise handelt es sich bei dem Dummy um ein konkretes medizinisches Operationsgerät, ggf. mit apparativer Peripherie zum Betreiben des Operationsgeräts. Insoweit sei nochmals auf die DE 10 2007 045 875 A1 verwiesen.

Ebenso kann es sich um einen nachgeahmten Fußschalter handeln, wie er bei Augenoperationen zum Bedienen des Handgeräts bzw. zur Ansteuerung der Peripherie zur Versorgung des Handgeräts verwendet wird. In weiter vorteilhafter Weise ist der Dummy über ein Kabel und eine USB-Schnittstelle mit dem PC verbunden, ähnlich, wie das tatsächliche Handgerät mit einer Antriebs-/Versorgungseinheit verbunden ist.

Das Handgerät und/oder der Fußschalter simulieren zumindest die Grundfunktionen des echten Geräts, wobei das Operationsprogramm auf eine entsprechende Betätigung sowie auf eine Handhabung des Handgeräts reagiert und die diesbezüglichen geometrischen Daten in die Simulation hineinrechnet und damit die Operationssituation anpasst.

In weiter vorteilhafter Weise werden das Operationsfeld und der Dummy 3D-animiert dargestellt, wobei es denkbar ist, dass die Darstellung in der Ansicht, insbesondere im Betrachtungswinkel, veränderbar ist. So lässt sich die Operation aus verschiedenen Blickwinkeln bzw. Perspektiven verfolgen, insbesondere auch unter dem Betrachtungswinkel des Operateurs.

In weiter vorteilhafter Weise lassen sich die einzelnen Operationsschritte protokollieren, wobei es weiter denkbar ist, dass der gesamte Operationsverlauf und ggf. das Operationsergebnis - automatisch - analysiert und der Trainingsperson mitgeteilt werden.

In ganz besonders vorteilhafter Weise dient das Operationsprogramm zur Anleitung zu einer spezifischen Operation, wonach zu den einzelnen Operationsschritten - nach Wahl - visuell und/oder per Sprache angeleitet wird. So ist es beispielsweise denkbar, dass einzelne Operationsschritte vom Operationsprogramm analysiert und anschließend von der Trainingsperson nachempfunden werden. Eine sprachliche Anleitung über die einzelnen Operationsschritte hinweg ist ebenfalls denkbar.

Auch ist es möglich, dass die einzelnen Operationsschritte - nach Wahl - per Sprache oder schriftlich kommentiert werden. Dabei können Verbesserungsvorschläge unterbreitet werden. In erfindungsgemäßer Weise wird das Ergebnis der simulierten Operation der Trainingsperson mit einem idealen Operationsergebnis - Schritt für Schritt - verglichen. Eine anschließende Analyse kann Fehler in den Operationsschritten aufdecken.

In weiter erfindungsgemäßer Weise werden der Trainingsperson Verbesserungsvorschläge in Bezug auf die einzelnen Operationsschritte aus dem Operationsprogramm mitgeteilt.

In ganz besonders raffinierter Weise wird die gesamte Aufzeichnung der virtuellen Operation an einen zentralen Rechner übermittelt. Im Falle des Zugriffs auf das Operationsprogramm über das Internet wird die virtuelle Operation - von vornherein - auf dem zentralen Rechner gespeichert und kann - nach Wahl der Trainingsperson - von einem über den Dienstleister des Operationsprogramms zur Verfügung gestellten Experten - zeitgleich, zeitnah oder zeitversetzt - ausgewertet und kommentiert werden. Auch hier können Verbesserungsvorschläge unterbreitet werden.

Zuvor ist bereits erwähnt worden, dass es sich bei der im Konkreten simulierten Operation beispielsweise um eine Catarakt-Operation am menschlichen Auge handeln kann. Grundsätzlich ist es denkbar, dass aus einem Eingangsmenü die Art der virtuellen Operation von der Trainingsperson auswählbar ist, wobei hier eine Auswahl von Operationstypen zur Verfügung gestellt wird, so beispielsweise dem Ophthalmologen verschiedene Operationstypen, die am menschlichen Auge durchzuführen sind, bis hin zu Korrekturen diverser Sehschwächen.

Die Nutzung des hier in Rede stehenden Operationsprogramms kann entgeltliche erfolgen, wobei dazu ein besonderes Abrechnungssystem zur Verfügung gestellt wird. Die der Trainingsperson entstehenden Kosten können von der Anzahl der unterschiedlichen virtuellen Operationen und der Nutzungsdauer abhängig sein.

Grundsätzlich ist es denkbar, dass der Anschluss des jeweiligen Dummy, d.h. des nachgeahmten Geräts an den Computer eine Vorauswahl der in Frage kommenden Operationen bedingt. Gleichzeitig ist es denkbar, dass über das angeschlossene Gerät eine partielle Freischaltung des Programms erfolgt.

Ebenso ist es denkbar, dass die Zugangsberechtigung zu dem Operationsprogramm über einen Hardwareschutz, vorzugsweise über einen am PC anzubringenden Dongel, bereitgestellt wird. Regelmäßig wird ein solcher Dongel über eine USB-Schnittstelle an den Computer angeschlossen, ebenso wie der Dummy. Dabei ist es von ganz besonderem Vorteil, wenn dem Dummy der Hardwareschutz zugeordnet ist bzw. ein Dummy implementiert werden kann, so dass mit der Ankopplung des Dummy eine oder mehrere konkrete virtuelle Operationen - automatisch - freigeschaltet werden.

Wie bereits zuvor erwähnt, ist die Trainingszeit und/oder die Anzahl der Zugriffe auf das Operationsprogramm limitiert. Vorzugsweise über das Internet lässt sich die Trainingszeit und/oder die Anzahl der Zugriffe entgeltlich erweitern, wobei dazu beliebige bekannte Abrechnungssysteme nutzbar sind. Auch ist es denkbar, dass der Trainingsperson die mit den Trainingseinheiten verbundene Kosten auf den späteren Kauf eines Handgeräts angerechnet werden oder dass mit dem Kauf eines Handgeräts ein gewisses Budget an Trainingseinheiten zur Verfügung gestellt wird.

Schließlich sei angemerkt, dass die voranstehenden Ausführungen lediglich der beispielhaften Erläuterung des erfindungsgemäßen Verfahrens dienen, dieses jedoch nicht auf die beispielhaften Ausführungen einschränken.

## Patentansprüche

1. Verfahren zur Durchführung einer virtuellen Operation zu Trainingszwecken, insbesondere eines virtuellen ophthalmologischen Eingriffs, wobei das Verfahren über ein Operationsprogramm definiert ist und folgende Verfahrensschritte umfasst, die über einen Computer, vorzugsweise über einen PC, von einer Trainingsperson dezentral abarbeitbar sind:
- Darstellung eines Operationsfeldes am menschlichen oder tierischen Körper, insbesondere eines Auges, auf einem Display,
- virtuelle Vornahme eines spezifischen Eingriffs mit mindestens einem einem konkreten medizinischen Operationsgerät, ggf. mit apparativer Peripherie, nachgeahmten Dummy, wobei der Dummy bzw. die Bewegung und die Betätigung des Dummy in das Operationsfeld hinein projiziert werden,
- interaktive Darstellung der Operationsschritte bzw. des Operationsergebnisses auf dem Display,
- Vergleich des Ergebnisses der simulierten Operation der Trainingsperson - Schritt für Schritt - mit einem idealen Operationsergebnis,
- Mitteilung von Verbesserungsvorschlägen in Bezug auf die einzelnen Operationsschritte aus dem Operationsprogramm,
wobei das Operationsprogramm über einen Datenträger oder über das Internet bereitgestellt wird und anhand einer Zugangsberechtigung von dem Datenträger ladbar bzw. über das Internet herunterladbar und aktivierbar ist und wobei der Anschluss des jeweiligen Dummy an den Computer eine Vorauswahl der in Frage kommenden Operationen bedingt.

2. Verfahren nach Anspruch 1, **dadurch gekennzeichnet, dass** es sich bei dem Dummy um ein nachgeahmtes chirurgisches Handgerät und/oder um einen nachgeahmten Fußschalter handelt.

3. Verfahren nach Anspruch 2, **dadurch gekennzeichnet, dass** das Handgerät und/oder der Fußschalter zumindest die Grundfunktionen des echten Geräts simuliert.

4. Verfahren nach einem der Ansprüche 1 bis 3, **dadurch gekennzeichnet, dass** das Operationsfeld und der Dummy 3D-animiert dargestellt werden und/oder in der Ansicht, insbesondere im Betrachtungswinkel, veränderbar sind.

5. Verfahren nach einem der Ansprüche 1 bis 4, **dadurch gekennzeichnet, dass** die einzelnen Operationsschritte protokolliert werden und der Operationsverlauf und ggf. das Operationsergebnis analysiert und der Trainingsperson mitgeteilt wird.

6. Verfahren nach einem der Ansprüche 1 bis 5, **dadurch gekennzeichnet, dass** zu den einzelnen Operationsschritten - nach Wahl - visuell und/oder per Sprache angeleitet wird.

7. Verfahren nach einem der Ansprüche 1 bis 6, **dadurch gekennzeichnet, dass** zu den einzelnen Operationsschritten - nach Wahl - per Sprache oder schriftlich kommentiert wird und ggf. Verbesserungsvorschläge unterbreitet werden.

8. Verfahren nach einem der Ansprüche 1 bis 7, **dadurch gekennzeichnet, dass** die gesamte Aufzeichnung der virtuellen Operation an einen zentralen Rechner übermittelt wird und - insbesondere bei Zugriff über das Internet - auf diesem gespeichert wird und - nach Wahl - von einem Experten ausgewertet und kommentiert wird.

9. Verfahren nach einem der Ansprüche 1 bis 8, **dadurch gekennzeichnet, dass** aus einem Eingangsmenu die Art der virtuellen Operation von der Trainingsperson auswählbar ist.

10. Verfahren nach einem der Ansprüche 1 bis 9, **dadurch gekennzeichnet, dass** die Zugangsberechtigung über einen Hardwareschutz, vorzugsweise über einen Dongel, bereitgestellt wird.

11. Verfahren nach einem der Ansprüche 1 bis 9, **dadurch gekennzeichnet, dass** die Zugangsberechtigung über den Dummy, welcher vorzugsweise über eine USB-Schnittstelle an den Computer angeschlossen wird, bereitgestellt wird.

12. Verfahren nach einem der Ansprüche 1 bis 11, **dadurch gekennzeichnet, dass** die Trainingszeit und/oder die Anzahl der Zugriffe auf das Operationsprogramm limitiert ist/sind.

13. Verfahren nach einem der Ansprüche 1 bis 12, **dadurch gekennzeichnet, dass** die Trainingszeit und/oder die Anzahl der Zugriffe über das Internet entgeltlich erweiterbar ist.

## Claims

1. Method for carrying out a virtual operation for training purposes, in particular virtual ophthalmological surgery, wherein the method is defined by an operation program and comprises the following method steps, which can be decentrally executed by a trainee by means of a computer, preferably by means of a PC:
- displaying an operating field for a human or animal body, in particular an eye, on a display,
- virtually carrying out a particular surgical procedure using at least one specific medical operating device, optionally using peripheral equipment, an imitation dummy, wherein the dummy or the movement and actuation of the dummy is projected into the operating field,
- interactively displaying the operation steps or the operation result on the display,
- comparing the result of the simulated operation carried out by the trainee, step by step, with an ideal operation result,
- providing suggestions for improvement regarding the individual operation steps from the operation program,
wherein the operation program is provided via a data medium or via the Internet and can be loaded from the data medium using an access authorisation or can be downloaded and activated via the Internet, and wherein the connection of the relevant dummy to the computer requires preselection of the operations in question.

2. Method according to claim 1, **characterised in that** the dummy is an imitation handheld surgical device and/or an imitation foot switch.

3. Method according to claim 2, **characterised in that** the handheld device and/or the foot switch simulates at least the basic functions of a real device.

4. Method according to one of claims 1 to 3, **characterised in that** the operating field and the dummy are displayed in 3D animation and/or the view thereof, in particular the viewing angle, can be altered.

5. Method according to one of claims 1 to 4, **characterised in that** the individual operation steps are logged and the progress of the operation and optionally the operation result are analysed and the trainee is given feedback.

6. Method according to one of claims 1 to 5, **characterised in that** instructions are given for the individual operation steps, according to choice, visually and/or by voice.

7. Method according to one of claims 1 to 6, **characterised in that** comments are made and optionally suggestions for improvements are given in relation to the individual operation steps, according to choice, by voice or in writing.

8. Method according to one of claims 1 to 7, **characterised in that** the entire recording of the virtual operation is transmitted to a central computer and, in particular when access is made via the Internet, is saved thereon and, according to choice, is evaluated and commented on by an expert.

9. Method according to one of claims 1 to 8, **characterised in that** the type of virtual operation can be selected by the trainee from an input menu.

10. Method according to one of claims 1 to 9, **characterised in that** the access authorisation is provided via a hardware protection system, preferably via a dongle.

11. Method according to one of claims 1 to 9, **characterised in that** the access authorisation is provided via the dummy, which is preferably connected to the computer via a USB interface.

12. Method according to one of claims 1 to 11, **characterised in that** the training time and/or the number of times the operation program is accessed are/is limited.

13. Method according to one of claims 1 to 12, **characterised in that** the training time can be extended and/or the number of times access is made via the Internet can be increased against a fee.

## Revendications

1. Procédé de réalisation d'une opération virtuelle à des fins de formation, plus particulièrement d'une intervention ophtalmologique virtuelle, le procédé étant défini par un programme d'opération et comprenant les étapes suivantes, qui sont exécutées par l'intermédiaire d'un ordinateur, de préférence par l'intermédiaire un PC, de manière décentralisée par une personne en formation :
- représentation d'un champ opératoire sur un corps humain ou animal, plus particulièrement d'un oeil, sur un écran,
- réalisation virtuelle d'une intervention spécifique avec au moins un dispositif d'opération médicale réel, le cas échéant avec des appareils périphériques, un mannequin, le mannequin ou le mouvement et l'actionnement du mannequin étant projeté à l'intérieur du champ opératoire,
- représentation interactive des étapes de l'opération ou du résultat de l'opération sur l'écran,
- comparaison du résultat de l'opération simulée de la personne en formation, étape par étape, avec un résultat d'opération idéal,
- notification de propositions d'amélioration concernant les différentes étapes de l'opération provenant du programme de l'opération,
le programme de l'opération étant mis à disposition par l'intermédiaire d'un support de données ou par l'intermédiaire d'Internet et pouvant être chargé à partir du support de données et téléchargé sur Internet et activé à l'aide d'une autorisation d'accès et la connexion du mannequin à l'ordinateur conditionnant une pré-sélection des opérations envisagées.

2. Procédé selon la revendication 1, **caractérisé en ce que** le mannequin est un appareil chirurgical imité et/ou une pédale imitée.

3. Procédé selon la revendication 2, **caractérisé en ce que** l'appareil portatif et/ou la pédale simule au moins les fonctions de base de l'appareil réel.

4. Procédé selon l'une des revendications 1 à 3, **caractérisé en ce que** le champ opératoire et le mannequin sont représentés de manière animée en 3D et/ou leur visualisation peut être modifiée, plus particulièrement en ce qui concerne l'angle de vue.

5. Procédé selon l'une des revendications 1 à 4, **caractérisé en ce que** les différentes étapes de l'opération sont consignées et le déroulement de l'opération et, le cas échéant, le résultat de l'opération sont analysés et notifiés à la personne en formation.

6. Procédé selon l'une des revendications 1 à 5, **caractérisé en ce que** les différentes étapes de l'opération sont guidées, au choix, visuellement et/ou vocalement.

7. Procédé selon l'une des revendications 1 à 6, **caractérisé en ce que** les différentes étapes de l'opération sont commentées, au choix, vocalement, ou par écrit et, le cas échéant, des propositions d'améliorations sont indiquées.

8. Procédé selon l'une des revendications 1 à 7, **caractérisé en ce que** l'ensemble de l'enregistrement de l'opération virtuelle est transmis à un ordinateur central et, plus particulièrement lors d'un accès par Internet, stocké sur celui-ci et, au choix, analysé et commenté par un expert.

9. Procédé selon l'une des revendications 1 à 8, **caractérisé en ce que**, à partir d'un menu d'entrée, le type d'opération virtuelle peut être sélectionné par la personne en formation.

10. Procédé selon l'une des revendications 1 à 9, **caractérisé en ce que** l'autorisation d'accès est effectuée par l'intermédiaire d'une protection matérielle, de préférence par l'intermédiaire d'un dongle.

11. Procédé selon l'une des revendications 1 à 9, **caractérisé en ce que** l'autorisation d'accès est effectuée par l'intermédiaire du mannequin, qui est connecté à l'ordinateur de préférence par l'intermédiaire d'une interface USB.

12. Procédé selon l'une des revendications 1 à 11, **caractérisé en ce que** le temps de formation et/ou le nombre d'accès au programme de l'opération sont limités.

13. Procédé selon l'une des revendications 1 à 12, **caractérisé en ce que** le temps de formation et/ou de nombre d'accès par l'intermédiaire d'Internet peuvent être étendus contre rémunération.
